# EUROPEAN PATENT SPECIFICATION

(11) **EP 2 967 227 B1**
(45) Date of publication and mention of the grant of the patent: **07.03.2018**
(21) Application number: 14720355.8
(22) Date of filing: 13.03.2014
(51) Int. Cl.: A47C 27/08, A47C 31/12, A61B 5/11, A61B 5/00, A47C 31/00, A61B 5/08

(54) **INFLATABLE AIR MATTRESS SLEEP ENVIRONMENT ADJUSTMENT AND SUGGESTIONS**
SCHLAFUMGEBUNGSANPASSUNG FÜR EINE AUFBLASBARE LUFTMATRATZE UND VORSCHLÄGE
RÉGLAGE ET SUGGESTIONS D'ENVIRONNEMENT DE SOMMEIL DE MATELAS PNEUMATIQUE GONFLABLE

(30) Priority: 14.03.2013 US 201361781571 P
(43) Date of publication of application: 20.01.2016
(73) Proprietor: SELECT COMFORT CORPORATION, Minneapolis, Minnesota 55442 (US); SleepIQ Labs Inc., San Jose CA 95113 (US)
(72) Inventor: NUNN, Rob, Eden Prairie, MN 55346 (US); PALASHEWSKI, Wade, Daniel, Minneapolis, MN 55442 (US); TILSTRA, Matthew, Wayne, Rogers, MN 55374 (US); STUSYNSKI, Stacy, Blaine, MN 55434 (US); YOUNG, Steven, San Jose,CA 95113 (US); HEWITT, Carl, San Jose, CA 95113 (US)
(74) Representative: Conroy, John
(86) International application number: PCT/US2014/026390
(87) International publication number: WO 2014/151753

(56) References cited:
- US-A- 5 948 303
- US-A1- 2005 143 617
- US-A1- 2008 052 837
- US-A1- 2010 170 043
- US-A1- 2011 015 495
- US-A1- 2012 112 891

## Description

### CROSS-REFERENCES

This Application claims the benefit of priority to U.S. Provisional Application No. 61/781,571, filed on March 14, 2013.

The subject matter described in this application is related to subject matter disclosed in the following applications: U.S. Application Serial No. 61/781,266 (Attorney Docket No. 3500.049PRV), filed on March 14, 2013, entitled "INFLATABLE AIR MATTRESS ALARM AND MONITORING SYSTEM"; U.S. Application Serial No. 61/781,503 (Attorney Docket No. 3500.050PRV), filed on March 14, 2013, entitled "INFLATABLE AIR MATTRESS SYSTEM ARCHITECTURE"; U.S. Application Serial No. 61/781,541 (Attorney Docket No. 3500.051PRV), filed on March 14, 2013, entitled "INFLATABLE AIR MATTRESS AUTOFILL AND OFF BED PRESSURE ADJUSTMENT"; U.S. Application Serial No. 61/782,394 (Attorney Docket No. 3500.053PRV), filed on March 14, 2013, entitled "INFLATABLE AIR MATTRESS SNORING DETECTION AND RESPONSE"; U.S. Application Serial No. 61/781,296 (Attorney Docket No. 3500.054PRV), filed on March 14, 2013, entitled "INFLATABLE AIR MATTRESS WITH LIGHT AND VOICE CONTROLS"; U.S. Application Serial No. 61/781,311 (Attorney Docket No. 3500.055PRV), filed on March 14, 2013, entitled "INFLATABLE AIR MATTRESS SYSTEM WITH DETECTION TECHNIQUES."

### TECHNICAL FIELD

This patent document pertains generally to a computer-readable medium including instructions for monitoring an inflatable air mattress system architecture.

### BACKGROUND

In various examples, an air mattress control system allows a user to adjust the firmness or position of an air mattress bed. The mattress may have more than one zone thereby allowing a left and right side of the mattress to be adjusted to different firmness levels. Additionally, the bed may be adjustable to different positions. For example, the head section of the bed may be raised up while the foot section of the bed stays in place. In various examples, two separate remote controls are used to adjust the position and firmness, respectively.

US 20080052837 describes a digital bed system that is comprised of an array of support cells. Each support cell is capable of communicating with a controller and increasing and decreasing in firmness in response to commands issued by a controller. The support cells are operatively connected to a communication channel that is also connected to a controller. The controller is capable of receiving data from the support cells and is also capable of issuing commands to each of the support cells. The controller is programmed issue commands to increase or decrease the firmness of individual support cells within the support cells.

### BRIEF DESCRIPTION OF DRAWINGS

**FIG. 1** is a diagrammatic representation of an air bed system, according to an example.
**FIG. 2** is a block diagram of various components of the air bed system of FIG. 1, according to an example.
**FIG. 3** is a block diagram of an air bed system architecture, according to an example
**FIG. 4** is a flow diagram of an example method (400) of defining and activating a sleep profile.
**FIG. 5** is a flow diagram depicting an example method (500) of detecting a sleep state or restfulness of a user
**FIG. 6** is a flow diagram of an example method (600) to recommend a sleep profile to a user
**FIG. 7** is a flow diagram of an example method of automatically adjusting a bed for a user
**FIG. 8** is a block diagram of machine in the example form of a computer system within which a set instructions, for causing the machine to perform any one or more of the methodologies discussed herein, may be executed.

### DETAILED DESCRIPTION

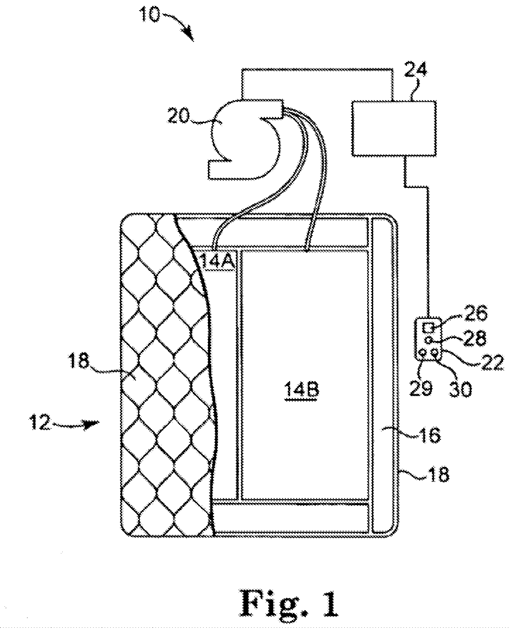

FIG. 1 is a diagrammatic representation of air bed system 10. System 10 may include bed 12, which may comprise at least one air chamber 14 surrounded by a resilient border 16 and encapsulated by bed ticking 18. The resilient border 16 may comprise any suitable material, such as foam.

As illustrated in FIG. 1, bed 12 may be a two chamber design having a first air chamber 14A and a second air chamber 14B. First and second air chambers 14A and 14B may be in fluid communication with pump 20. Pump 20 may be in electrical communication with a remote control 22 via control box 24. Remote control 22 may communicate via wired or wireless means with control box 24. Control box 24 may be configured to operate pump 20 to cause increases and decreases in the fluid pressure of first and second air chambers 14A and 14B based upon commands input by a user through remote control 22. Remote control 22 may include display 26, output selecting means 28, pressure increase button 29, and pressure decrease button 30. Output selecting means 28 may allow the user to switch the pump output between the first and second air chambers 14A and 14B, thus enabling control of multiple air chambers with a single remote control 22. For example, output selecting means may by a physical control (e.g., switch or button) or an input control displayed on display 26. Alternatively, separate remote control units may be provided for each air chamber and may each include the ability to control multiple air chambers. Pressure increase and decrease buttons 29 and 30 may allow a user to increase or decrease the pressure, respectively, in the air chamber selected with the output selecting means 28. Adjusting the pressure within the selected air chamber may cause a corresponding adjustment to the firmness of the air chamber.

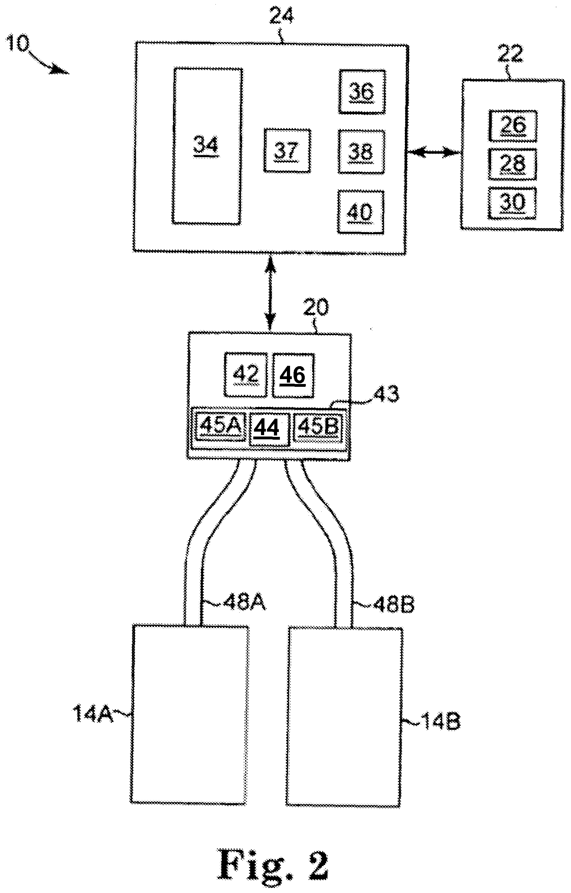

FIG. 2 is a block diagram detailing data communication between certain components of air bed system 10 according to various examples. As shown in FIG. 2, control box 24 may include power supply 34, processor 36, memory 37, switching means 38, analog to digital (A/D) converter 40, and radios for communication with remotes and smartphones. Switching means 38 may be, for example, a relay or a solid state switch. Switching means 38 may be located in the pump 20 rather than the control box 24.

Pump 20 and remote control 22 may be in two-way communication with the control box 24. Pump 20 may include a motor 42, a pump manifold 43, a relief valve 44, a first control valve 45A, a second control valve 45B, and a pressure transducer 46, and may be fluidly connected with the first air chamber 14A and the second air chamber 14B via a first tube 48A and a second tube 48B, respectively. First and second control valves 45A and 45B may be controlled by switching means 38, and may be operable to regulate the flow of fluid between pump 20 and first and second air chambers 14A and 14B, respectively.

In an example, pump 20 and control box 24 may be provided and packaged as a single unit. Alternatively, pump 20 and control box 24 may be provided as physically separate units.

In operation, power supply 34 may receive power, such as 110 VAC power, from an external source and may convert the power to various forms required by certain components of the air bed system 10. Processor 36 may be used to control various logic sequences associated with operation of the air bed system 10, as will be discussed in further detail below.

The example of the air bed system 10 shown in FIG. 2 contemplates two air chambers 14A and 14B and a single pump 20. However, other examples may include an air bed system having two or more air chambers and one or more pumps incorporated into the air bed system to control the air chambers. In an example, a separate pump may be associated with each air chamber of the air bed system or a pump may be associated with multiple chambers of the air bed system. Separate pumps may allow each air chamber to be inflated or deflated independently and simultaneously. Furthermore, additional pressure transducers may also be incorporated into the air bed system such that, for example, a separate pressure transducer may be associated with each air chamber.

In the event that the processor 36 sends a decrease pressure command to one of air chambers 14A or 14B, switching means 38 may be used to convert the low voltage command signals sent by processor 36 to higher operating voltages sufficient to operate relief valve 44 of pump 20 and open control valves 45A or 45B. Opening relief valve 44 may allow air to escape from air chamber 14A or 14B through the respective air tube 48A or 48B. During deflation, pressure transducer 46 may send pressure readings to processor 36 via the A/D converter 40. The A/D converter 40 may receive analog information from pressure transducer 46 and may convert the analog information to digital information useable by processor 36. Processor 36 may send the digital signal to remote control 22 to update display 26 on the remote control in order to convey the pressure information to the user.

In the event that processor 36 sends an increase pressure command, pump motor 42 may be energized, sending air to the designated air chamber through air tube 48A or 48B via corresponding valve 45A or 45B. While air is being delivered to the designated air chamber in order to increase the firmness of the chamber, pressure transducer 46 may sense pressure within pump manifold 43. Again, pressure transducer 46 may send pressure readings to processor 36 via A/D converter 40. Processor 36 may use the information received from A/D converter 40 to determine the difference between the actual pressure in air chamber 14A or 14B and the desired pressure. Processor 36 may send the digital signal to remote control 22 to update display 26 on the remote control in order to convey the pressure information to the user.

Generally speaking, during an inflation or deflation process, the pressure sensed within pump manifold 43 provides an approximation of the pressure within the air chamber. An example method of obtaining a pump manifold pressure reading that is substantially equivalent to the actual pressure within an air chamber is to turn off pump 20, allow the pressure within the air chamber 14A or 14B and pump manifold 43 to equalize, and then sense the pressure within pump manifold 43 with pressure transducer 46. Thus, providing a sufficient amount of time to allow the pressures within pump manifold 43 and chamber 14A or 14B to equalize may result in pressure readings that are accurate approximations of the actual pressure within air chamber 14A or 14B. In various examples, the pressure of 48A/B is continuously monitored using multiple pressure sensors.

In an example, another method of obtaining a pump manifold pressure reading that is substantially equivalent to the actual pressure within an air chamber is through the use of a pressure adjustment algorithm. In general, the method may function by approximating the air chamber pressure based upon a mathematical relationship between the air chamber pressure and the pressure measured within pump manifold 43 (during both an inflation cycle and a deflation cycle), thereby eliminating the need to turn off pump 20 in order to obtain a substantially accurate approximation of the air chamber pressure. As a result, a desired pressure setpoint within air chamber 14A or 14B may be achieved without the need for turning pump 20 off to allow the pressures to equalize. The latter method of approximating an air chamber pressure using mathematical relationships between the air chamber pressure and the pump manifold pressure is described in detail in U.S. Application Serial No. 12/936,084.

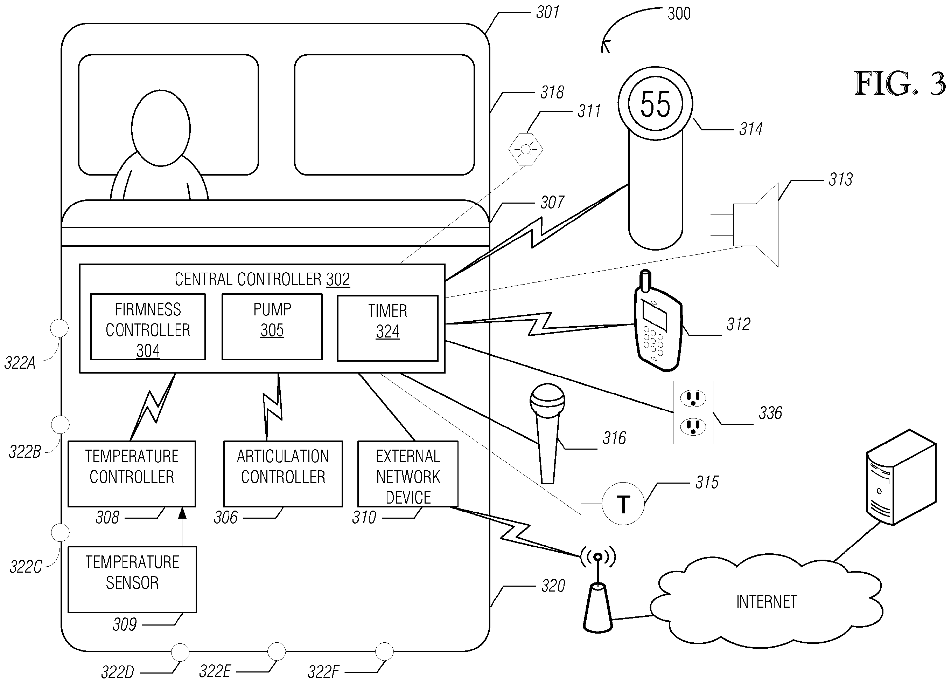

FIG. 3 is illustrates an example air bed system architecture 300. Architecture 300 includes bed 301, central controller 302, firmness controller 304, articulation controller 306, temperature controller 308, external network device 310, remote controllers 312, 314, and voice controller 316. While described as using an air bed, the system architecture may also be used with other types of beds.

As illustrated in FIG. 3, network bed architecture 300 is configured as a star topology with central controller 302 and firmness controller 304 functioning as the hub and articulation controller 306, temperature controller 308, external network device 310, remote controls 312, 314, and voice controller 316 functioning as possible spokes, also referred to herein as components. Thus, in various examples, central controller 302 acts a relay between the various components.

In other examples, different topologies may be used. For example, the components and central controller 302 may be configured as a mesh network in which each component may communicate with one or all of the other components directly, bypassing central controller 302. In various examples, a combination of topologies may be used. For example, remote controller 312 may communicate directly to temperature controller 308 but also relay the communication to central controller 302.

In yet another example, central controller 302 listens to communications (*e.g*., control signals) between components even if the communication is not being relayed through central controller 302. For example, consider a user sending a command using remote 312 to temperature controller 308. Central controller 302 may listen for the command and check to determine if instructions are stored at central controller 302 to override the command (*e.g*., it conflicts with a previous setting). Central controller 302 may also log the command for future use (*e.g*., determining a pattern of user preferences for the components).

In various examples, the controllers and devices illustrated in FIG. 3 may each include a processor, a storage device, and a network interface. The processor may be a general purpose central processing unit (CPU) or application-specific integrated circuit (ASIC) (referred to collectively as processor(s)). The storage device may include volatile or non-volatile static storage (e.g., Flash memory, RAM, EPROM, etc.). The storage device may store instructions which, when executed by the processor, configure the processor to perform the functionality described herein. For example, a processor of firmness control 304 may be configured to send a command (*e.g*., signal) to a relief valve to decrease the pressure in a bed.

In various examples, the network interface of the components may be configured to transmit and receive communications in a variety of wired and wireless protocols. For example, the network interface may be configured to use the 802.11 standards (e.g., 802.11a/b/c/g/n/ac), PAN network standards such as 802.15.4 or Bluetooth, infrared, cellular standards (e.g., 3G/4G etc.), Ethernet, and USB for receiving and transmitting data. The previous list is not intended to exhaustive and other protocols may be used. Not all components of FIG. 3 need to be configured to use the same protocols. For example, remote control 312 may communicate with central controller 302 via Bluetooth while temperature controller 308 and articulation controller 306 are connected to central controller using 802.15.4. Within FIG. 3, the lightning connectors represent wireless connections and the solid lines represent wired connections, however, the connections between the components is not limited to such connections and each connection may be wired or wireless.

Moreover, in various examples, the processor, storage device, and network interface of a component may be located in different locations than various elements used to effect a command. For example, as in FIG. 1, firmness controller 302 may have a pump that is housed in a separate enclosure than the processor used to control the pump. Similar separation of elements may be employed for the other controllers and devices in FIG. 3.

In various examples, firmness controller 304 is configured to regulate pressure in an air mattress. For example, firmness controller 304 may include a pump such as described with reference to FIG. 2 (see e.g., pump 20). Thus, in an example, firmness controller 304 may respond to commands to increase or decrease pressure in the air mattress. The commands may be received from another component or based on stored application instruction that are part of firmness controller 304.

As illustrated in FIG. 3 central controller 302 includes firmness controller 304. Thus, in an example, the processor of central controller 302 and firmness control 304 may be the same processor. Furthermore, pump 305 may also be part of central controller 302. Accordingly, central controller 302 may be responsible for pressure regulation as well as other functionality as described in further portions of this disclosure.

In various examples, articulation controller 306 is configured to adjust the position of a bed *(e.g.,* bed 301) by adjusting foundation 307 that supports the bed. In an example, bed 301 may include a single foundation 307 configured to adjust the position of a bed having a single mattress. In another example, bed 301 may include two side-by-side foundations 307 configured to operate in tandem to adjust the position of a bed having a single mattress. In yet another example, bed 301 may include two side-by-side mattresses supported by two side-by-side foundations 307, wherein the foundations 307 are operable independently such that separate positions may be set for the two different mattresses of the bed 301. Foundation 307 may include more than one zone, *e.g.,* head portion 318 and foot portion 320, which may be independently adjusted. Articulation controller 306 may also be configured to provide different levels of massage to a person on the bed.

In various examples, temperature controller 308 is configured to increase, decrease, or maintain the temperature of a user. For example, a pad may be placed on top of or be part of the air mattress. Air may be pushed through the pad and vented to cool off a user of the bed. Conversely, the pad may include a heating element that may be used to keep the user warm. In various examples, temperature controller 308 receives temperature readings from the pad.

In various examples, additional controllers may communicate with the central controller 302. These controllers may include, but are not limited to, illumination controllers for controlling the power status (e.g., on or off) or intensity of light elements 311 and 322A-F placed on and around the bed, audio/visual controllers for controlling the power status or volume of one or more audio/visual components 313 located near the bed, thermostat controllers for controlling a temperature setting of a thermostat device 315, and outlet controllers for controlling power to one or more power outlets 336.

In various examples, external network device 310, remote controllers 312, 314 and voice controller 316 may be used to input commands *(e.g.,* from a user or remote system) to control one or more components of architecture 300. The commands may be transmitted from one of the controllers 312, 314, or 316 and received in central controller 302. Central controller 302 may process the command to determine the appropriate component to route the received command. For example, each command sent via one of controllers 312, 314, or 316 may include a header or other metadata that indicates which component the command is for. Central controller 302 may then transmit the command via central controller 302's network interface to the appropriate component.

For example, a user may input a desired temperature for the user's bed into remote control 312. The desired temperature may be encapsulated in a command data structure that includes the temperature as well as identifies temperature controller 308 as the desired component to be controlled. The command data structure may then be transmitted via Bluetooth to central controller 302. In various examples, the command data structure is encrypted before being transmitted. Central controller 302 may parse the command data structure and relay the command to temperature controller 308 using a PAN. Temperature controller 308 may be then configure its elements to increase or decrease the temperature of the pad depending on the temperature originally input into remote control 312.

In an example implementation, central controller 302 may detect user presence using temperature changes detected in the mattress, e.g., using one or more temperature sensors positioned in or on the mattress. The temperature sensors and the central controller 302 may detect a rise in temperature, e.g., over a specified period of time, and determine that a user is present in the bed. For example, if central controller 302 detects a rise in temperature and then determines that the detected rise in temperature was not caused by the system's temperature controller 308, the central controller 302 may determine that the user is present.

In various examples, data may be transmitted from a component back to one or more of the remote controls. For example, the current temperature as determined by a sensor element of temperature controller 308, the pressure of the bed, the current position of the foundation or other information may be transmitted to central controller 302. Central controller 302 may then transmit the received information and transmit it to remote control 312 where it may be displayed to the user.

In various examples, multiple types of devices may be used to input commands to control the components of architecture 300. For example, remote control 312 may be a mobile device such as a smart phone or tablet computer running an application. Other examples of remote control 312 may include a dedicated device for interacting with the components described herein. In various examples, remote controls 312/314 include a display device for displaying an interface to a user. Remote control 312/314 may also include one or more input devices. Input devices may include, but are not limited to, keypads, touchscreen, gesture, motion and voice controls.

Remote control 314 may be a single component remote configured to interact with one component of the mattress architecture. For example, remote control 314 may be configured to accept inputs to increase or decrease the air mattress pressure. Voice controller 316 may be configured to accept voice commands to control one or more components. In various examples, more than one of the remote controls 312/314 and voice controller 316 may be used.

With respect to remote control 312, the application may be configured to pair with one or more central controllers. For each central controller, data may be transmitted to the mobile device that includes a list of components linked with the central controller. For example, consider that remote control 312 is a mobile phone and that the application has been authenticated and paired with central controller 302. Remote control 312 may transmit a discovery request to central controller 302 to inquiry about other components and available services. In response, central controller 302 may transmit a list of services that includes available functions for adjusting the firmness of the bed, position of the bed, and temperature of the bed. The application may then display functions for increasing/decreasing pressure of the air mattress, adjusting positions of the bed, and adjusting temperature. If components are added/removed to the architecture under control of central controller 302, an updated list may be transmitted to remote control 312 and the interface of the application may be adjusted accordingly.

In various examples, central controller 302 is configured as a distributor of software updates to components in architecture 300. For example, a firmware update for temperature controller 308 may become available. The update may be loaded into a storage device of central controller 302 (e.g., via a USB interface or wireless techniques). In wireless applications, the central controller 302 may, for example, receive updates from the cloud either from Wi-Fi or from a mobile connection over Bluetooth. Central controller 302 may then transmit the update to temperature controller 308 with instructions to update. Temperature controller 308 may attempt to install the update. A status message may be transmitted from temperature controller 308 to central controller 302 indicating the success or failure of the update.

In various examples, central controller 302 is configured to analyze data collected by a pressure transducer (e.g., transducer 46 with respect to FIG. 2) to determine various states of a person lying on the bed. For example, central controller 302 may determine the heart rate or respiration rate of a person lying in the bed. Additional processing may be done using the collected data to determine a possible sleep state of the person. For example, central controller 302 may determine when a person falls asleep and, while asleep, the various sleep states of the person.

In various example, external network device 310 includes an network interface to interact with an external server for processing and storage of data related to components in architecture 300. For example, the determined sleep data as described above may be transmitted via a network (e.g., the Internet) from central controller 302 to external network device 310 for storage. In an example, the pressure transducer data may be transmitted to the external server for additional analysis. The external network device 310 may also analyze and filter the data before transmitting it to the external server.

In an example, diagnostic data of the components may also be routed to external network device 310 for storage and diagnosis on the external server. For example, if temperature controller 308 detects an abnormal temperature reading (e.g., a drop in temperature over one minute that exceeds a set threshold) diagnostic data (sensor readings, current settings, etc.) may be wireless transmitted from temperature controller 308 to central controller 302. Central controller 302 may then transmit this data via USB to external network device 310. External device 310 may wirelessly transmit the information to an WLAN access point where it is routed to the external server for analysis.

In various examples, architecture 300 includes a feature to adjust multiple components of the bed based on a time (*e.g.,* a countdown or specific time) or event trigger (*e.g.,* a sleep state of the user). For example, a profile may be defined that includes settings for multiple components of architecture 300. The settings may include, but are not limited to, a pressure setting for one or more mattresses controlled by firmness controller 304, a position setting for one or more foundations 307 controlled by articulation controller 306, a temperature of one or more pads controlled by temperature controller 308, illumination of light elements 322A-F controlled by an illumination controller, and power settings for one or more outlets 336 controlled by an outlet controller. The profile is referred to herein as a sleep profile, but other labels may be used. Similarly, other labels of buttons and UI elements used herein are for illustration proposes and other labels may be used without departing from the scope of this disclosure. While the profile is used with respect to sleep in the examples that follow, similar profiles could be established for use during the day or when a user wakes up from sleep.

In an example, the sleep profile may be a data structure that includes one or more tuples of a feature of a component of architecture 300, a setting for the feature, and a trigger (*e.g*., "{Pressure; 65; One hour after initial activation}, {Temperature; 72; One hours after REM sleep has been achieved}"). In various examples, instead of/or in addition to having individual triggers, the sleep profile may include a global trigger. The sleep profile may be defined by the user alone or in combination with recommendations based on past sleep patterns of the user (stored in central controller 302 or on an external server) as discussed further below. Additionally, multiple sleep profiles may be defined that are associated with different triggers.

In various examples, triggers are time based, event based, or a combination of the two. In an example, the time may be an absolute time such as "9:00 PM on Fridays" or the time may be relative to the activation of the sleep profile - one hour after activation (*e.g*., the user selected a UI element on controller 312, 314, or 316 representing the sleep profile). Event based triggers may be associated with a sleep state of the user. For example, the event may be that the user has fallen asleep or is in a particular state of sleep. A combination trigger may be defined such that a profile may be activated one hour after detecting the user has fallen asleep.

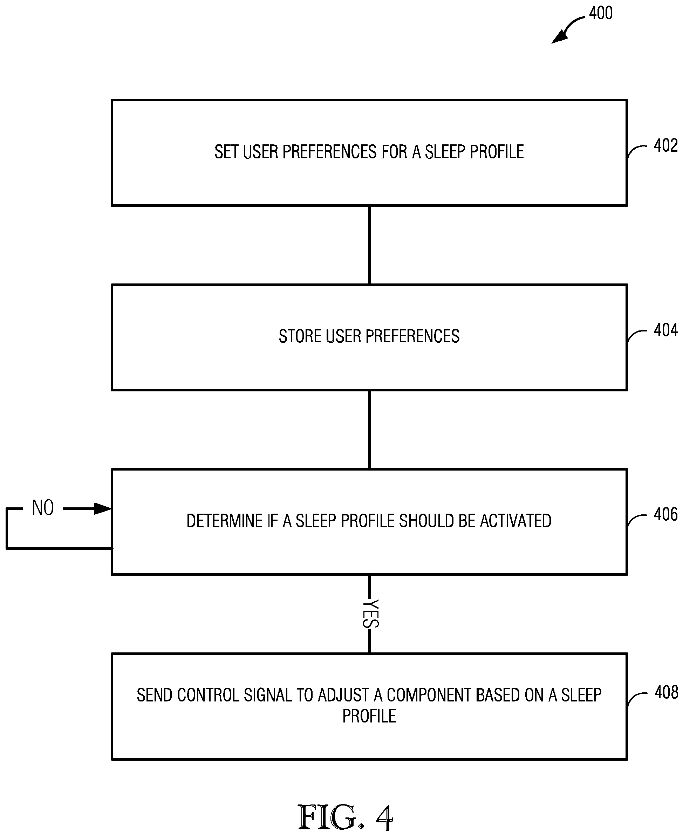

**FIG. 4** illustrates a flow diagram of an example method (400) of defining and activating a sleep profile. To establish a profile, user preferences for the sleep profile may be set using one or more of controllers 312, 314, and 316 (402). For example, using an application running on smart phone 312, a user interface (UI) may be presented to the user. The UI may present an option to a user to define a new sleep profile or modify an existing sleep profile. In an example, recommendations to modify or create a new sleep profile are presented based on previous monitoring of a user's restfulness during sleep.

Upon receiving an indication that the user is creating a new sleep profile, a UI may presented with input indicia (check boxes, radio buttons, input forms, etc.) for settings of components that may be used in a sleep profile. For example, central controller 302 may maintain a list of components that are communicatively coupled with central controller 302 and present available settings for the components. In this manner, settings for components that are not part of architecture 300 (*e.g.,* if a user does not have an adjustable foundation) are not presented to the user.

In various examples, a user may interact (*e.g*., click, activate) with the input indicia to indicate the settings to use for the various components and an associated trigger. The input indicia may be configured to present a range of available options for each component. For example, an input box may be presented to a user for setting a pressure of a bed with accompanying text that states "Please enter a number of 35-100." In addition to the settings for the components, a user may optionally title the sleep profile. After a user has entered in settings for one or more of the available components a user may select a "Save Settings" button to indicate the user is done entering in settings.

In various examples, a sleep profile may be defined to another level of granularity allowing a user to identify multiple settings for a component over the course of a night. For example, a user may indicate that the temperature of a pad should be 72 degrees upon initial activation of the sleep profile, but should drop to 68 degrees an hour after REM sleep has been achieved.

After the preferences (*e.g*., settings, title, triggers) for a profile have been established, the preferences may be transmitted to central controller 302 and stored in a storage device (404). In various examples, the preferences may be stored in a database (relational, non-relational, flat file, etc.) or in a structured file (e.g., XML).

In a scenario where a user is modifying an existing sleep profile, central controller may retrieve a list of existing sleep profiles as stored in a storage device of central controller 302. For example, sleep profiles may be stored according to title or a numbering system within a storage device of central controller 302. Thus, a UI may be presented (*e.g*., on a smart phone app of remote control 312) to a user that include the titles of each stored sleep profile. The user may then select a sleep profile from the list, an indication of the selection may be transmitted to central controller 302, and central controller 302 may retrieve the preferences associated with the selected sleep profile. Accordingly, the UI may be updated to include the retrieved the stored preferences for the sleep profile. The user may then update the sleep profile, and upon completing any changes, select the "Save Settings" button. Upon activation of the button, the updated settings may be transmitted back to central controller 302, which then updates the stored sleep profile on the storage device.

In various examples, instructions executing on central controller 302 determine if a sleep profile should be activated (406). Determining may include checking if a sleep profile is current enabled. For example, in addition to identifying settings for a sleep profile, a user may indicate whether the sleep profile is active or inactive. In an example, only one sleep profile may be enabled at a time to avoid conflicts between sleep profile settings. For an enabled sleep profile, central controller 302 may be determine if the sleep profile should be activated based on the sleep profiles associated triggers or manual activation by a user (*e.g*., central controller 302 receives an indication from a controller that a user has activated a sleep profile). As indicated previously, each component of architecture 300 may have a different trigger in a sleep profile. Accordingly, activation of a sleep profile may be a complete activation or partial activation. Flow continues back to 406 if it is determined that the conditions for triggering an activate of a sleep profile have not been met.

For time based triggers, central controller 302 may utilize timer 324 to determine if a sleep profile should be activated. For sleep cycle based on triggers, central controller 302 may monitor the sleep cycle state of a user by analyzing pressure readings throughout the night collected through one or more pressure sensors (*e.g*., pressure transducer 46).

For example, system architecture 300 may detect biometric parameters of a user such as motion, respiration, and heartbeat via pressure sensor readings. These biometric parameters may be detected both while the user is awake and while the user is sleeping. In various examples, the biometric parameters may be used to determine a sleep state of the user. Techniques for monitoring a user's sleep using heart rate information, respiration rate information, and other user information are disclosed in U.S. Patent Application Publication No. 20100170043 to Steven J. Young et al., titled "APPARATUS FOR MONITORING VITAL SIGNS".

In accordance with this disclosure, central controller 302 may detect user sleeping motion, respiration, and heartbeat via pressure changes. For example, pressure transducer 46 (of **FIG. 2**) may be used to monitor the air pressure in the air mattress of the bed 301. If the user on the air mattress is not moving, the air pressure changes in the mattress may be relatively minimal, and may be attributable to respiration and heartbeat. When the user on the air mattress is moving, however, the air pressure in the mattress may fluctuate by a much larger amount. Thus, the pressure signals generated by the pressure transducer 46 and received by the central controller 302 may be filtered and indicated as corresponding to motion, heartbeat, or respiration.

In an example implementation, central controller 302 may execute instructions that cause the pressure transducer 46 to measure air pressure values at a predefined sample rate. The central controller 302 may store the pressure signals in a memory device. Processing of the pressure signals may be performed by central controller 302, or at a location remote from bed 301. Analyzing the pressure signals, as indicated above, central controller 302 may determine a user's sleep state, *e.g*., rapid eye movement ("REM") or non-rapid eye movement ("NREM"), by using one or more of the biometric parameters..

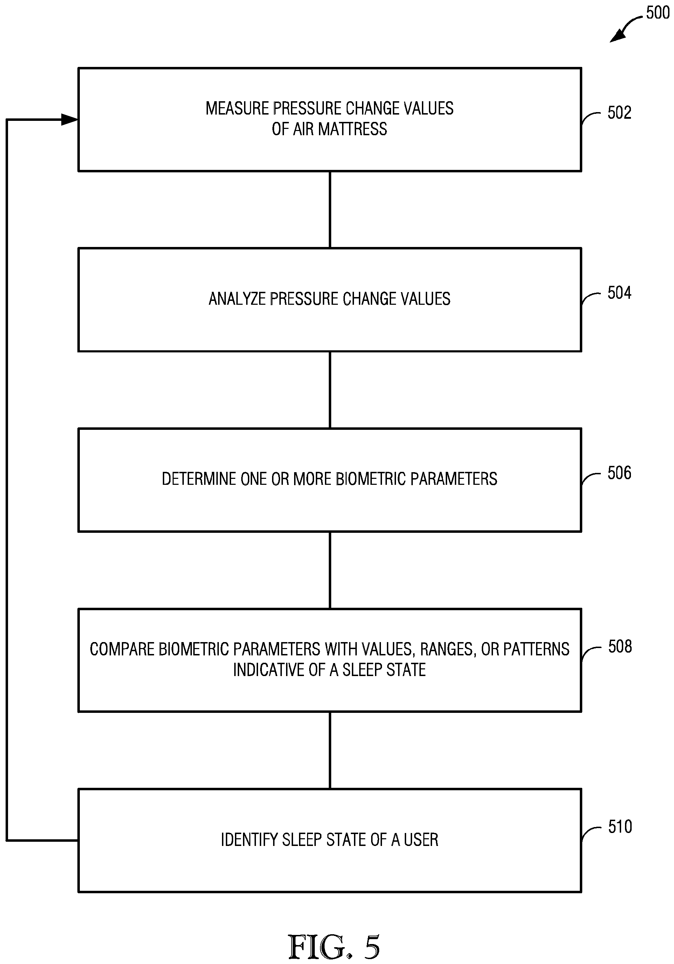

**FIG. 5** is a flow diagram depicting an example method (500) of detecting a sleep state or restfulness of a user. In an example, this method is used to determine if the conditions of an event based trigger have been met. This method may also be used for recommending changes to a sleep profile or establishing a sleep pattern of a user as further discussed below.

In **FIG. 5**, central controller 302 executes instructions that cause a pressure sensing means, such as the pressure transducer 46 of FIG. 2, to measure pressure variations (502). In an example, the pressure may be measured continuously or at a predetermined sample rate. The central controller 302 may analyze the pressure changes detected by the pressure sensing means (504). Using information derived from these analyzed pressure changes, one or more biometric parameters may be determined (506). After determining the one or more biometric parameters of the user, the central controller 302 may compare the biometric parameters with predetermined values, ranges, or patterns which are indicative of a particular sleep state or restfulness of the user (508). In this manner, the central controller 302 may identify the sleep state of a user or how well the user is sleeping (510).

Returning back to **FIG. 4**, upon determining that a sleep profile should be activated, control signals may be sent to one or more controllers to adjust components of architecture 300 according to the sleep profile (408). For example, if a trigger is that one hour after REM sleep has been achieved, the temperature of a pad should be reduced to 68 degrees the following process may be used. Central controller 302 may first determine that a user has entered REM sleep using a method such as described in FIG. 5. Then, central controller 302 may start a count-down timer of one hour using timer 324. Central controller 302 may receive an indication (*e*.*g*., signal) from timer 324 that the hour has passed. Then, central controller 324 may transmit a control signal to temperature controller 308 to change the temperature of a pad to 68 degrees.

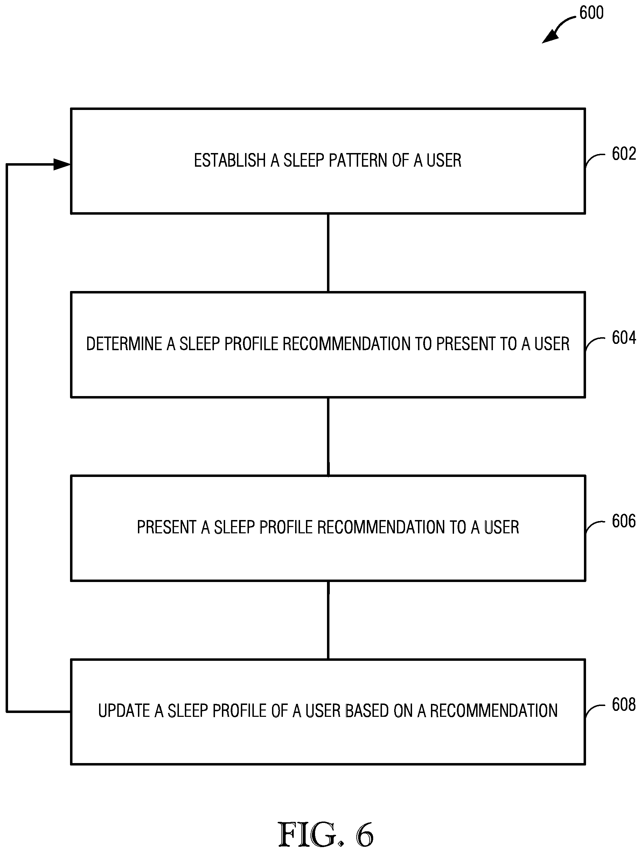

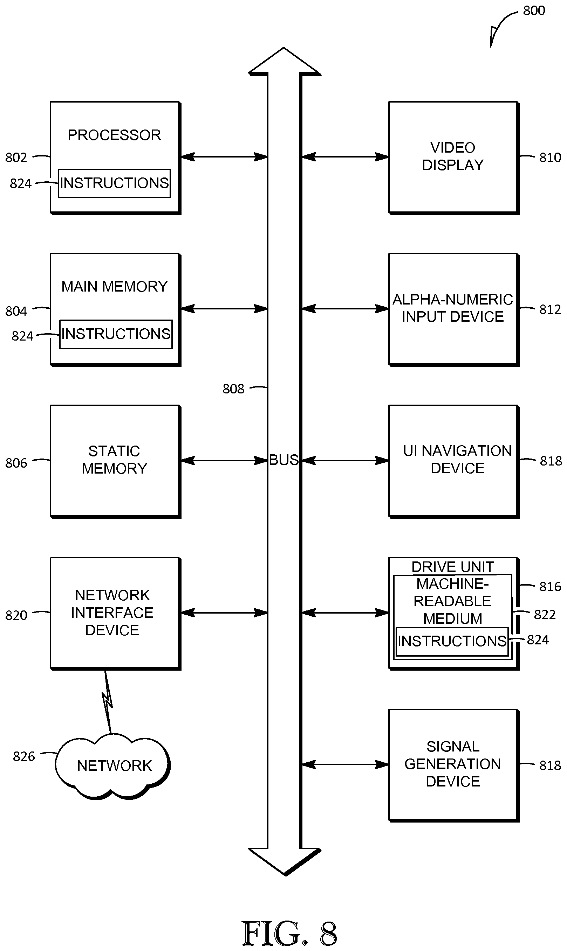

**FIG. 6** illustrates a flow diagram of an example method (600) to recommend a sleep profile to a user. In various embodiments, recommendations to a user may be provided to alter their sleep profiles, or if a sleep profile has not been created, to establish a sleep profile.
As discussed in regard to FIG. 8, a machine-readable medium 822 may store one or more sets of instructions and data structures (e.g., software) 824 embodying or utilized by this methodology.

In an example, central controller 302 establishes a sleep pattern for a user (602). A sleep pattern may include an index of restfulness of the user throughout the night. For example, restfulness may be determined according to the ratio of REM or deep sleep a user is getting throughout the night compared to total sleep time as determined using a method as described in FIG. 5. Other definitions of restfulness may also be used (*e.g.,* the amount of movement - as sensed by or more pressure sensors - a user for a given night as compared to the average amount of movement).

In an example, central controller 302 determines a recommendation to present to a user (604). A sleep pattern may include multiple entries of restfulness and associated settings of components of architecture 300 when the entry was recorded. Accordingly, central controller 302 may analyze the entries to determine which settings are associated with more or less restfulness (e.g., using a regression analysis or other statistical techniques). For example, central controller 302 may determine that when, all other settings being equal, the pressure of the bed is "50," the biometric signals of the user indicate that the user is less restful than a pressure setting of "60." Therefore, the recommendation may be for the user to have the bed adjust to a setting of "60" after a sleep state has been achieved. Multiple recommendations may be made and stored at central controller 302. In various examples, based on the above analysis, a ranking of which settings correlate strongest with more restful sleep may be generated and displayed to a user.

In an example, recommendation is presented to the user (606). For example, when a user begins to create or modify an existing sleep profile via a remote control, a query may be transmitted to central controller 302 requesting any stored recommendations. The recommendations, if any, may be transmitted back to the remote control and presented to the user. In an example, in addition to the recommendation, an option may be presented to the user to accept the recommendation for one or more sleep profiles.

In further examples, sleep patterns of many users may be aggregated at an external server (*e.g*., by respective central controllers transmitting anonymous sleep data to the server). The aggregated data may be analyzed to determine a sleep recommendation for the user. This analysis may be done, for example, at the external server. The recommendation may be received via external network device 310 and stored in a similar fashion as the individualized recommendation. In various examples, recommendations based on the aggregate data are presented to the user instead of the individualized recommendation or in addition to the individualized recommendations.

In an example, a sleep profile of a user is updated based on the recommendation (608). For example, using the method described in FIG. 4, a sleep profile may be updated at central controller 302 with any settings of the recommendation that the user has indicated to accept.

In an example, instead of a sleep profile being created for the user that is editable and viewable by the user, architecture 300 may include a feature which, if enabled by the user, automatically adjusts the components of architecture 300 during the sleep cycle.

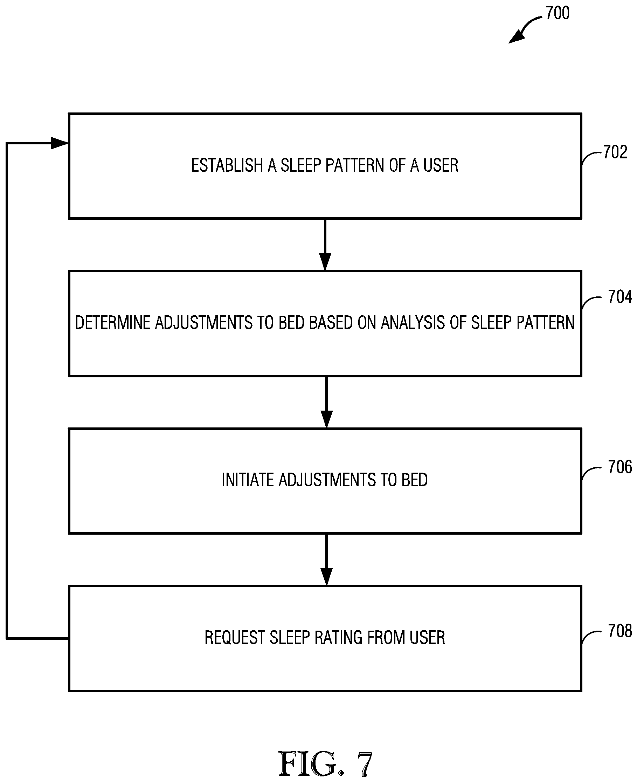

**FIG. 7** is a flow diagram of an example method of automatically adjusting a bed for a user.
As discussed in regard to FIG. 8,
a machine-readable medium 822 may store one or more sets of instructions and data structures (e.g., software) 824 embodying or utilized by this methodology.

In an example, a sleep pattern of a user is established (702) as discussed previously (602). Central controller 302 may then determine one or more adjustments to make to a component of the bed based on an analysis of the sleep pattern of a user (704). For example, the adjustments may be the similar to the recommendations discussed in FIG. 6. During the next night, the adjustments may be made to the components (706) upon determining the user is asleep or by manual activation by a user. In various examples, a UI may be presented the following morning asking the user to rate the previous night's sleep (708) via a remote control. Central controller 302 may use this information in addition to the biometric information of the sleep pattern to make further adjustments to enhance the user's sleep experience the next night with flow returning to block 702.

### EXAMPLE MACHINE ARCHITECTURE AND MACHINE-READABLE MEDIUM

**FIG. 8** is a block diagram of machine in the example form of a computer system 800 within which instructions, for causing the machine to perform any one or more of the methodologies discussed herein, may be executed. In alternative embodiments, the machine operates as a standalone device or may be connected (e.g., networked) to other machines. In a networked deployment, the machine may operate in the capacity of a server or a client machine in server-client network environment, or as a peer machine in a peer-to-peer (or distributed) network environment. The machine may be a personal computer (PC), a tablet PC, a set-top box (STB), a Personal Digital Assistant (PDA), a cellular telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein.

The example computer system 800 includes a processor 802 (e.g., a central processing unit (CPU), a graphics processing unit (GPU), ASIC or a combination), a main memory 804 and a static memory 806, which communicate with each other via a bus 808. The computer system 800 may further include a video display unit 810 (e.g., a liquid crystal display (LCD) or a cathode ray tube (CRT)). The computer system 800 also includes an alphanumeric input device 812 (e.g., a keyboard), a user interface (UI) navigation device 814 (e.g., a mouse), a disk drive unit 816, a signal generation device 818 (e.g., a speaker) and a network interface device 820.

### MACHINE-READABLE MEDIUM

The disk drive unit 816 includes a machine-readable medium 822 on which is stored one or more sets of instructions and data structures (e.g., software) 824 embodying or utilized by any one or more of the methodologies or functions described herein. The instructions 824 may also reside, completely or at least partially, within the main memory 804 and/or within the processor 802 during execution thereof by the computer system 800, the main memory 804 and the processor 802 also constituting machine-readable media.

While the machine-readable medium 822 is shown in an example embodiment to be a single medium, the term "machine-readable medium" may include a single medium or multiple media (e.g., a centralized or distributed database, and/or associated caches and servers) that store the one or more instructions or data structures. The term "machine-readable medium" shall also be taken to include any tangible medium that is capable of storing, encoding or carrying instructions for execution by the machine and that cause the machine to perform any one or more of the methodologies of the present invention, or that is capable of storing, encoding or carrying data structures utilized by or associated with such instructions. The term "machine-readable medium" shall accordingly be taken to include, but not be limited to, solid-state memories, and optical and magnetic media. Specific examples of machine-readable media include non-volatile memory, including by way of example semiconductor memory devices, e.g., Erasable Programmable Read-Only Memory (EPROM), Electrically Erasable Programmable Read-Only Memory (EEPROM), and flash memory devices; magnetic disks such as internal hard disks and removable disks; magneto-optical disks; and CD-ROM and DVD-ROM disks.

### TRANSMISSION MEDIUM

The instructions 824 may further be transmitted or received over a communications network 826 using a transmission medium. The instructions 824 may be transmitted using the network interface device 820 and any one of a number of well-known transfer protocols (e.g., HTTP). Examples of communication networks include a local area network ("LAN"), a wide area network ("WAN"), the Internet, mobile telephone networks, Plain Old Telephone (POTS) networks, and wireless data networks (e.g., WiFi and WiMax networks). The term "transmission medium" shall be taken to include any intangible medium that is capable of storing, encoding or carrying instructions for execution by the machine, and includes digital or analog communications signals or other intangible media to facilitate communication of such software.

## Claims

1. A non-transitory computer-readable medium including instructions, which when executed by at least one processor, cause the at least one processor to:
establish (602, 702) a sleep pattern of a user of a bed architecture by monitoring biometric parameters of the user during sleep and calculating a restfulness index of the user;
determine (604, 704), using a central controller (302) of the bed architecture, an adjustment to make to a component of the bed architecture based on the sleep pattern by determining a pressure setting adjustment for an air mattress of the bed architecture;
present (606) the adjustment of the component as a recommendation to the user;
initiate (706) the adjustment to the component; and
request (708) feedback from the user with respect to the initiated adjustment.

2. The non-transitory computer-readable medium of claim 1, wherein the instructions further cause the at least one processor to determine an adjustment to make to a component of the bed architecture based on the sleep pattern by:
determining a temperature setting adjustment for a pad of the bed architecture.

3. The non-transitory computer-readable medium of claim 1, wherein the instructions further cause the at least one processor to:
determine another adjustment to make to a component of the bed architecture based on the feedback.

4. The non-transitory computer-readable medium of claim 1, wherein the instructions further cause the at least one processor to display to the user a ranking of which settings correlate strongest with more restful sleep.

5. The non-transitory computer-readable medium of claim 1, wherein the instructions further cause the at least one processor to:
when a user begins to create or modify an existing sleep profile via a remote control, transmit a query to the central controller (302) requesting any stored recommendations; and
present any recommendations that are transmitted back to the user.

6. The non-transitory computer-readable medium of claim 1, wherein the instructions further cause the at least one processor to, in addition to the recommendation, present an option to the user to accept a recommendation for one or more sleep profiles.

7. The non-transitory computer-readable medium of claim 1, wherein the instructions further cause the at least one processor to update a sleep profile with a setting of the recommendation that the user has indicated to accept.

## Patentansprüche

1. Nichttransitorisches computerlesbares Medium mit Anweisungen, die, wenn sie von mindestens einem Prozessor ausgeführt werden, bewirken, dass der mindestens eine Prozessor Folgendes durchführt:
Herstellen (602, 702) eines Schlafmusters eines Benutzers einer Bettarchitektur durch Überwachen von biometrischen Parametern des Benutzers während des Schlafs und Berechnen eines Erholsamkeitsindex des Benutzers;
Bestimmen (604, 704) einer Anpassung, die an einer Komponente der Bettarchitektur vorzunehmen ist, auf Basis des Schlafmusters unter Verwendung einer zentralen Steuerung (302) der Bettarchitektur durch Bestimmen einer Druckeinstellungsanpassung für eine Luftmatratze der Bettarchitektur;
Präsentieren (606) der Anpassung der Komponente als eine Empfehlung für den Benutzer;
Initiieren (706) der Anpassung der Komponente und
Anfordern (708) einer Rückmeldung vom Benutzer mit Bezug auf die initiierte Anpassung.

2. Nichttransitorisches computerlesbares Medium nach Anspruch 1, wobei die Anweisungen ferner bewirken, dass der mindestens eine Prozessor auf Basis des Schlafmusters eine Anpassung bestimmt, die durch Folgendes an der Bettarchitektur vorzunehmen ist:
Bestimmen einer Temperatureinstellungsanpassung für eine Unterlage der Bettarchitektur.

3. Nichttransitorisches computerlesbares Medium nach Anspruch 1, wobei die Anweisungen ferner bewirken, dass der mindestens eine Prozessor Folgendes durchführt:
Bestimmen auf Basis der Rückmeldung einer weiteren Anpassung, die an einer Komponente der Bettarchitektur vorzunehmen ist.

4. Nichttransitorisches computerlesbares Medium nach Anspruch 1, wobei die Anweisungen ferner bewirken, dass der mindestens eine Prozessor dem Benutzer eine Rangfolge dazu anzeigt, welche Einstellungen am stärksten mit erholsamerem Schlaf korrelieren.

5. Nichttransitorisches computerlesbares Medium nach Anspruch 1, wobei die Anweisungen ferner bewirken, dass der mindestens eine Prozessor Folgendes durchführt:
wenn ein Benutzer beginnt, ein bestehendes Schlafprofil über eine Fernbedienung zu erstellen oder zu modifizieren, Übertragen einer Anfrage zur zentralen Steuerung (302) zum Anfordern gespeicherter Empfehlungen und
Präsentieren von Empfehlungen, die zum Benutzer zurück übertragen werden.

6. Nichttransitorisches computerlesbares Medium nach Anspruch 1, wobei die Anweisungen ferner bewirken, dass der mindestens eine Prozessor dem Benutzer zusätzlich zu der Empfehlung eine Option präsentiert, eine Empfehlung für ein oder mehrere Schlafprofile zu akzeptieren.

7. Nichttransitorisches computerlesbares Medium nach Anspruch 1, wobei die Anweisungen ferner bewirken, dass der mindestens eine Prozessor ein Schlafprofil mit einer Einstellung der Empfehlung aktualisiert, von der der Benutzer angezeigt hat, dass er sie akzeptiert.

## Revendications

1. Support lisible par ordinateur non transitoire incluant des instructions, qui lorsqu'elles sont exécutées par au moins un processeur, amènent ledit au moins un processeur à :
établir (602, 702) un schéma de sommeil d'un utilisateur d'une architecture de lit grâce à la surveillance des paramètres biométriques de l'utilisateur durant le sommeil et au calcul d'un indice de repos de l'utilisateur ;
déterminer (604, 704), grâce à l'utilisation d'un contrôleur central (302) de l'architecture de lit, un ajustement à réaliser sur un composant de l'architecture de lit basé sur le schéma de sommeil en déterminant un ajustement du réglage de pression pour un matelas pneumatique de l'architecture de lit ;
présenter (606) à l'utilisateur l'ajustement du composant à titre de recommandation ;
entreprendre (706) l'ajustement du composant ; et
demander (708) une réaction de la part de l'utilisateur par rapport à l'ajustement ayant été entrepris.

2. Support lisible par ordinateur non transitoire de la revendication 1, les instructions amenant en outre ledit au moins un processeur à déterminer un ajustement à réaliser sur un composant de l'architecture de lit. basé sur le schéma de sommeil en :
déterminant un ajustement du réglage de température pour une zone de l'architecture de lit.

3. Support lisible par ordinateur non transitoire de la revendication 1, les instructions amenant en outre ledit au moins un processeur à :
déterminer un autre ajustement à réaliser sur un composant de l'architecture de lit basé sur la réaction.

4. Support lisible par ordinateur non transitoire de la revendication 1, les instructions amenant en outre ledit au moins un processeur à afficher à l'intention de l'utilisateur un classement dont les réglages ont la plus forte corrélation avec un sommeil plus reposant.

5. Support lisible par ordinateur non transitoire de la revendication 1, les instructions amenant en outre ledit au moins un processeur à :
lorsqu'un utilisateur commence à créer ou modifier un profil de sommeil existant via une télécommande, transmettre une interrogation au contrôleur central (302) laquelle demande toutes les recommandations éventuelles stockées ; et
présenter toutes les recommandations éventuelles qui sont transmises en retour à l'utilisateur.

6. Support lisible par ordinateur non transitoire de la revendication 1, les instructions amenant en outre ledit au moins un processeur à, en plus de la recommandation, présenter une option à l'utilisateur pour accepter une recommandation pour un ou plusieurs profils de sommeil.

7. Support lisible par ordinateur non transitoire de la revendication 1, les instructions amenant en outre ledit au moins un processeur à mettre à jour un profil de sommeil avec un réglage de la recommandation que l'utilisateur a indiqué d'accepter.
